# EUROPEAN PATENT SPECIFICATION

(11) **EP 1 200 135 B1**
(45) Date of publication and mention of the grant of the patent: **17.12.2003**
(21) Application number: 00938076.7
(22) Date of filing: 02.06.2000
(51) Int. Cl.: A61L 2/28

(54) **MONITORING METHOD FOR STERILIZATION WITH ETHYLENE OXIDE**
KONTROLLVERFAHREN FUR DIE STERILISATION MIT ETHYLENOXID
METHODE DE CONTROLE DE LA STERILISATION AVEC DE L'OXYDE D'ETHYLENE

(30) Priority: 06.08.1999 US 138064 P
(43) Date of publication of application: 02.05.2002
(73) Proprietor: Patel, Gordhanbhai N., Middlesex, NJ 08846 (US)
(72) Inventor: Patel, Gordhanbhai N., Middlesex, NJ 08846 (US)
(74) Representative: Winter, Brandl, Fürniss, Hübner, Röss, Kaiser, Polte Partnerschaft
(86) International application number: US0015241
(87) International publication number: WO01010471

(56) References cited:
- WO-A-00/61200
- WO-A-98/58683
- US-A- 4 145 186
- US-A- 4 206 844
- CHEMICAL ABSTRACTS, vol. 81, no. 1, 8 July 1974 (1974-07-08) Columbus, Ohio, US; abstract no. 20, NAKAJIMA TADASHI: "ETHYLENE OXIDE STERILISATION DETECTION" page 425; XP002148519 & NAKAJIMA TADASHI: "Ethylene Oxide Sterilization Detection" JAPAN KOKAI, vol. 74, no. 49, 14 May 1974 (1974-05-14), page 693

## Description

### BACKGROUND OF THE INVENTION

### 1. FIELD OF THE INVENTION

The present invention relates to the use of a color changing chemical indicatior device for monitoring sterilization of medical supplies with ethylene oxide. The device undergoes at least one color change with time, temperature and concentration of ethylene oxide.

### 2. BRIEF DESCRIPTION OF PRIOR ART

Products, such as medical supplies are sterilized to kill living organisms to an acceptable level. Direct testing for sterility is destructive and expensive and hence indirect testing methods are used. Biological indicators made from cultures, such as Bacillus subtilis spores, bacilus pumilus spores and clostridium sporogenes spores are used for monitoring the sterilization. However, chemical indicators are widely used because they are simple and inexpensive.

A wide variety of medical supplies are sterilized with material and techniques, such as steam, plasma, high energy radiation and ethylene oxide. Ethylene oxide is abbreviated herein as ETO. It is essential to assure that the medical supply is sterilized. A number of indicators, dosimeters and monitors are proposed In the literature. They include biological and chemical indicators. The color changing chemical indicators are inexpensive and hence are widely used.
number of patents have been issued on ethylene oxide indicators.

U.S. Pat. No. 3.852.034 describes an indicator for ethylene oxide which includes an amino substituted indicating compound. e.g., acid salts of amino substituted triphenylmethanes. diphenylmethanes. azines or xanthenes. which undergoes color change based on replacement of labile hydrogen in amino groups with hydroxyethyl and a buffering agent selected to provide an ionic dissociation equilibrium such that color change occurs only when sterilization has been effective.

An indicator composition for ethylene oxide gas comprising 4-(4-nitrobenzyl)pyridine, nitrocellulose, a basic substance, and, optionally, a blue coloring agent is disclosed in U.S. Pat. No. 4,094,642. The indicator composition, when exposed to ethylene oxide gas, changes its color to develop a stable color indication, typically of a green color. The composition can take any shape and form, and is typically applied to a substrate, such as paper to form a layer thereon. Devices using 4-(4-nitrobenzyl)pyridine are described in U.S. Pat. No. 4,678,640, U.S. Pat. No. 4,826,772 and U.S. Pat. No. 4,436,819. For example U.S. Pat. No. 4,436,819 describes a device for colorimetrically quantifying exposure to ethylene oxide which comprises a polymer substrate which exhibits transport for ethylene oxide through the body of which is dispersed a concentration of a color-forming compound which undergoes a color-change upon reaction with ethylene oxide, said concentration being chosen to provide a variable degree of color-change in said device which, at a uniform temperature, is a previously determined function of the device's quantity of exposure to ethylene oxide. The color forming compound comprises a material selected from the group of ethylene oxide-reactive color formers consisting of 4-(p-nitrobenzyl)pyridine, N-phenylberizylpyridine and phenazine and the basic catalysts is a member selected from the group consisting of triethanolamine, triethylenediamine, triethylenetetramine, N,N-bis(aminopropyl)-1,3-propanediamine, and N,N,N',N'-tetrakis(methyl)-ethylenediamine.

An indicating composition, which undergoes a color change, that is progressive with the conditions and periods of sterilization, such that a final and complete color change indicates the completion of effective ethylene oxide sterilization is disclosed in U.S. Pat. No. 4,407,960. The indicating composition comprises a leuco precursor of an aryl methane dye selected from the groups of dyes such as Michler's hydrol, crystal violet lactone, malachite green leuco and crystal violet leuco; and an acidic constituent, such as diphenolic acid (4,4-bis [4-hydroxyphenyl] pentanoic acid).

A device for giving a visual indication of the amount of residual ethylene oxide present in ethylene oxide treated solid hospital articles and other health devices is disclosed in U.S. Pat. No. 4,495,291. The amount of residual ethylene oxide is indicated by the difference in color exhibited by different sensitized areas of the device in the form of an opaque dark colored support member having bonded to its surface a thin film of a cholesteric liquid crystal composition in a solid film-forming binder.

An ink composition for indicating the progress of sterilization with ethylene oxide is provided in U.S. Pat. No. 5,258,065 which comprises; (1) at least one disperse dye of the general formula A-N=N-B wherein A is a residue of a heterocyclic compound containing nitrogen atom which is not substituted with alkyl group and selected from the group consisting of the pyridine, quinoline, isoquinoline, triazole, tetrazole, indazole, thiazole, benzothiazole and thiadiazole rings, which residue may optionally have one or more undissociated substituents, and B is a coupling component, (2) at least one binder component selected from the group consisting of polyacrylic acid, polymethacrylic acid and acrylic acid-methacrylic acid copolymers, (3) at least one ultrafine filler selected from the group consisting of ultraline particles of silica, aluminum oxide and titanium oxide, and (4) at least one polar solvent.

An ink composition has been prepared as a telltale for ethylene oxide sterilization, which utilizes the fact that magnesium chloride reacts with ethylene oxide to produce a base. magnesium hydroxide, which is detected by a pH sensitive dye; see U.S. Pat. No. 3,093,751. A further development in this area of ethylene oxide monitoring is disclosed in U.S. PaL No. 4,138,216. The device disclosed comprises a wick impregnated with magnesium chloride and a pH sensitive dye is enclosed in a gas impervious envelope having one end open. An additional constituent is an acidic material, e.g., tartaric acid. which acts as a quantifier to adjust the time response of the device. This latter device is particularly useful in ethylene oxide sterilization monitoring because it is responsive to humidity levels as well as temperature and gas concentration. U.S. Pat. No. 5.451.372 discloses a device for monitoring an ethylene oxide sterilization process in which 100% ethylene oxide is utilized as the sterilant comprising a wick impregnated with an ethylene oxide responsive chemical compound a quantifier and a pH sensitive dye: an ethylene oxide impervious backing strip upon which the wick is mounted; and a cover strip having an ethylene oxide impervious film. The backing and cover strip are adhered to one another, and in intimate contact with and sealed to the wick. The ethylene oxide responsive chemical compound is a chloride of divalent metals such as magnesium, iron and zinc and the pH sensitive dye is bromophenol blue, thymol blue or xylenol blue.

Even though the production of a base upon exposure to ethylene oxide is reported in U.S. Pat. No. 3,098,751, 4,138,216 and 5451372, it is always limited to chlorides of divalent metals, such as magnesium, iron and zinc. There is no report on use of halides of (1) mono-valent metals, such as sodium and potassium, (2) the other halides. such as bromides or iodides, of di or higher valent metals. (3) organic halides, such as tetrabutylammonium bromides, for monitoring ethylene oxide and (4) other salts, both organic and inorganic, such as sodium thiocyanate. We have accidentally discovered that a coating composed of a monovalent organic or inorganic salt, such as sodium bromide, sodium thiocyanate and tetraethylammonium bromide, a pH sensitive dye, such as bromophenol blue, and a polymeric binder, such as a polyacrylate. undergoes a color change when exposed to ethylene oxide. WO 98/58 683 discloses a sterilization monitor including a substrate and a monitor composition. The monitor composition contains a colorant and a halogen source and undergoes a distinct color change when exposed to a peracid. The sterilization monitor can be used to monitor a sterilization process involving a peracid. WO 00/61 200 discloses indicators for monitoring sterilization with oxidizing. agents or plasma. Sterilization with ETO depends upon several factors, such as time. temperature, humidity and concentration of ETO. In order to assure the sterilization with ETO, the indicator . must determine integral value of these, it is also desirable that the indicator, in the absence of ethylene oxide, is essentially unaffected by other parameters, such as dry heat, steam. radiation and ambient conditions.

### SUMMARY OF THE INVENTION

There is provided a process using a device for monitoring sterilization of materials, said device comprising
at least one layer of polymer, having incorporated therein
a) an indicator capable of undergoing at least one color change when subjected to a rise in pH,
b) an activator for said indicator, said activator being a compound of the group consisting of organic and inorganic salts, metal chelates and metal complexes, having a monovalent cation, reactable with ethylene oxide, to cause a rise in pH said rise in pH causing said indicator to undergo said color change, comprising the steps of
c) affixing the device to said materials or containers containing same
d) carrying out the process of sterilization including the step of introducing ethylene oxide and
e) observing the presence of a color change of said device.

Such a device is made by coating a mixture of (a) a polymer as binder, (b) a ETO reactive salt having a monovalent cation and (c) a pH sensitive dye, when contacted with ETO, undergoes at least one color change. Such a device can be used for monitoring sterilization of medical supplies.

The formulations and devices disclosed herein offer several advantages over those based on magnesium chloride reported in U.S. Pal. No. 3,098,751, 4.138.216 and 5451372. The hydroxides of higher valent metals. such as magnesium hydroxide. are significantly weaker than those of alkali metals. such as potassium hydroxide and sodium hydroxide produced upon exposure to ethylene oxide. As a result, the device made from alkali metal and organic halides are significantly more sensitive than those made from magnesium chloride. Lower concentration of ethylene oxide can be detected with the devices disclosed herein. For example, a paper impregnated with sodium iodide or tetrabutylammonium bromide and bromothymol blue changes from yellow- to- blue almost instantly when exposed to 100% ethylene oxide or in minutes at about 20ppm of ethylene oxide. Hence, using the formulations proposed herein, one can make more sensitive devices similar to those proposed in U.S. Pat. No. 3.098.751. 4,138,216 and 5451372. As monovalent halides, such as sodium iodide and potassium bromide are generally less sensitive to humidity than those of higher valent metals, the device disclosed herein is more stable to environmental humidity and reasonably sensitive to a mixture of humidity and ethylene oxide compared to those made from magnesium chloride and calcium chloride. The change in pH upon exposure to ETO is higher in case of mono-valent halides and hence the color change is more dramatic and a larger number of pH sensitive dyes can be used to get a variety of color changes upon exposure to ethylene oxide. We have also found that di and higher vaiant metal halides cause precipitation of aqueous inks vehicle/binders, whicn are made water soluble or dispersible by incorporating acidic functionalities. Hence, it is very difficult to incorporate divalent metal salts sucn as magnesium chloride without causing the precipitation of most of the aqueous vehicles. The mono-valent salts usually have lower toxicity and prices compared to those of the di-valent metal salts.

The indicators suitable for use in this device include pigments. dyes, precursors of said dyes. and mixtures of any of these. A desirable quality of the indicator is the ability to undergo a color change with the reaction product of ethylene oxide and said activator. Desirably the indicator undergoes a yellow-to-blue, yellow-to-green, red-to-yellow red-to-green or red-to-blue color change.

The polymer used in the device is, suitably, soluble in water or dispersible in an aqueous medium solvent. A broad class of polymers may be used. They may be homopolymers. copolymers or a mixure thereof.

Suitably the reaction product of the activator and ethylene oxide is a base. Suitable activators are salts of monovalent cations, such as halides and isocyanates. preferably bromides. The activator may also be a salt of an amine and an organic or inorganic acid.

The device may additionally comprise an additive, such as an acid or base, to control the rate of reaction and color change.

The device may have two layers, that is to say additionally composing a polymeric top layer. This may be a wedge shaped polymeric top layer.

The process of making such a device comprises dissolving the components thereof in a solvent thereof, applying the thus formed solution to a substrate and permitting the solvent to evaporate.

The substrate may be a container for an item to be sterilized. It may also be a plastic film, paper or metal, including but not timited polyester film. paper or spun bonded polyolefins.

In a desirable embodiment of the invention the solution is an ink formulation suitably an aqueous ink formulation most suitably one which comprises an acrylate polymer.

### Embodiments of the present invention comprise:

A process of using a device for monitoring ethylene oxide said device comprising at least one layer of polymer, having incorporated therein
a) an indicator capable of undergoing at least one color change when subjected to a rise in pH.
b) an activator for said indicator, said activator being a compound of the group consisting of organic and inorganic salts, metal chelates and metal complexes, having a monovalent cation, reactable with ethylene oxide, to cause a rise in pH said rise in pH causing said indicator to undergo said color change, comprises the steps of
c) exposing the device to ethylene oxide
d) observing the presence of color change in the device.

A method for monitoring sterilization with ethylene oxide comprising:
exposing a monitor composition comprises at least one layer of polymer, having incorporated therein
   a) an indicator capable of undergoing at least one color change when subjected to a rise in pH
   b) an activator for said indicator, said activator being a compound of the group consisting of organic and inorganic salts, metal chelates and metal complexes, having a monovalent cation, reactable with ethylene oxide, to cause a rise in pH said rise in pH causing said indicator to undergo said color change
when exposed to ethylene oxide and
noting the change in color of said indicator.

### BRIEF DESCRIPTION OF THE DRAWINGS

Figure 1. A side schematic cross section of one embodiment of the ETO sterilization indicator used in the invention where an indicator layer comprised of a polymeric binder, ETO activator and ETO indicator is applied on a substrate.
Figure 2. A side schematic cross section of the ETO sterilization indicator used in the invention having an adhesive layer and a release layer.
Figure 3. A side cross-section of a multi-layer device wherein a top layer is a coating or lamination as a barrier.

### BRIEF DESCRIPTION OF THE INVENTION AND PREFERRED EMBODIMENTS

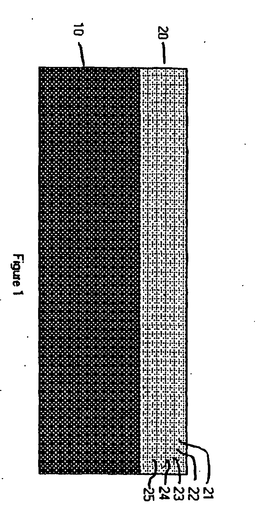

The device can be best described by reference to the Figures. As shown in Figure 1. the device in one of the simplest form is comprised of an indicator layer 20, applied on a substrate 10. The substrate 10 can also be a container, such as pouch for products to be sterilized. The indicator layer 20 is composed of a polymeric binder 21. containing at least one ETO indicator, such as a pH sensitive dye 22. capable of undergoing a color change with a reaction product such as a base. produced by reaction of ETO with the ETO activator 23. when contacted with ETO. The coating 20 may contain other additives, e.g.. additive 24 to control the rate of the color change and additive 25 to maintain the colors and shelf life.

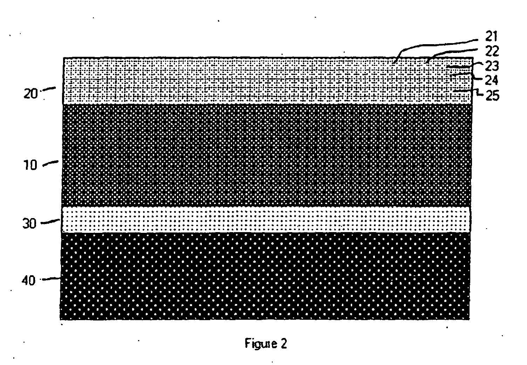

As shown in Figure 2. the substrate 10 of the device can be coated with an adhesive layer 30. The adhesive layer allows the device to be affixed to a container of product to be sterilized. To the bollom of the adhesive layer 30, can be affixed a release layer 40 for ease in packaging and for removal just prior to use. Removal of the release layer 40 will enable the entire device to be affixed to the container of product to be sterilized.

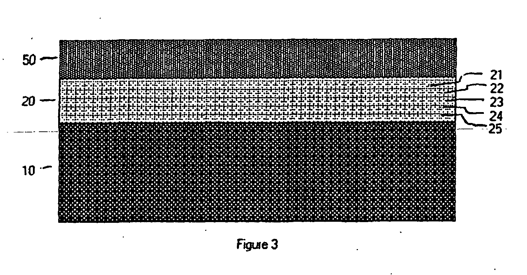

The device can be composed of more than one layer. In its simplest form, the device could have two layers. As shown in Figure 3, in its simplest form of the two-layer device, the second top layer 50 could be a barrier for ETO. e.g., a polymeric coat or a laminated film, on to layer 20. The barrier layer 50 can reduce diffusion of ETO, thereby increasing the time required for the color change.

A moving boundary device can be created if the barrier layer 50 is in form a wedge over the indicator layer 20. The barrier layer will resist but will be permeable to ethylene oxide.

Other variations of the ETO indicator device are also possible. for example, a gradient device can be created by coating a series of formulations having the time required for the color change either increases or decreases. Such gradient can be obtained by coating such formulations in form of lines or bars next to each other.

The device can also be created by printing the indicating formulation in form of a number, image, bar code or message, e.g., "if this print is green, the product inside is sterilized".

The invention has been practiced by using an acrylate printing ink extender 001270 supplied by Environmental Inks and Coating. Co. Lithicum, MO as a binder and tetraethylammonium bromide as an ETO activator and bromocresol purple as an indicator. The acrylate ink extender 001270 is referred herein to as EC001270 and tetraethylammonium bromide as TEAB.

Classes of dyes: A wide variety of dyes which change color with change in pH can be used as indicators, such as nitroso. nitro, azo (mono. di, tri and polyazo). azoic, stilbene, carotenoid, diphenylmethane. triphenylmethane, xanthene. acridine. quinoline, methane and polymethine. thiazole, indamine and indophenol, azine, oxazine, sulfur. lactone, aminoketone, hydroxyketone, anthraquinone, indigoid, phthalocyanine. and natural.

A dye having an amine functionality and neutralized with acids, such as HI and HBr can undergo color change with ETO without activator or would require lower concentration of activator.

Concentration of the activator required for the noticeable color change depends upon several factors, such as thickness of the coating and absorbance coefficient of the dyes. Preferred concentration is 0.1 to 20% of the total solid of the coating. The most preferred range of the indicator concentration is 0.5 to 5%.

A large number of dyes, as listed in Table 1, were added in a mixture of EC001270 (1) TEAB (2) sodium thiocyanate and (3) tetrabutylphosphonium bromide and coated on paper and polyester film. Pieces of the coatings were exposed to ETO.

Table 1. List of dyes tested with TEAB, sodium thiocyanate and tetrabutylphosphonium bromide as ETO activator in EC001270.

Acid alizarin violet N. acid black 24, acid black 48, acid blue 113, acid blue 120, acid blue 129. acid blue 161, acid blue 25, acid blue 29, acid blue 40, acid blue 41, acid blue 45, acid blue 80. acid blue 93, acid fuschin. acid green 25, acid green 27. acid green 41. acid orange 74, acid red 1, acid red 114, acid red 151, acid red 88, acid violet 17, acid violet 7, acid yellow 99, acridine orange, acridine orange base, acridine orange G, acridine yellow G, acriflavine hydrochloride, alcian blue 8GX, alcian yellow, alizarin, alizarin blue black SN, alizarin complexone, alizarin complexone dihydrate, alizarin red, alizarin violet 3R, alizarin yellow GG, alizarin yellow R, alkali blue 6B, alkali fast green 10GA, alphazurine A, aluminon, aminoacridine hydrochloride, aminoanthraquinone, aminophthalhydrazide, aniline blue, astra blue 6GLL, auramine O, azocarmine, azocarmine B, azure A, azure B, azure B thiocyanate, azure C, basic blue 3, basic blue 41, basic blue 66, basic fuchsin, basic red 29, basic yellow. 11, benzo purpurin 4B, biebrich scarlet NA salt, bismarck brown B, bismarck brown Y, blue tetrazolium, bordeaux R, brilliant blue B, brilliant blue G, brilliant cresyl blue ALD, brilliant crocein MOO, brilliant green, brilliant sulphaflavine, brilliant yellow, bromochlorophenol blue, bromocresol green, bromocresol purple, bromophenol blue, bromopyrogallol red, bromothymol blue, bromoxylenol blue, calmagite, carbol fuchsin, carminic acid, carotene, celestine blue, Chicago sky blue, chlorophenol red, chrome azurol S, chromotrope 2B, chromotrope 2R, chromoxane cyanine B, chrysoidin, chrysophenine, cibacron brilliant red 3BA, Congo red, copper(II) phthalocyanine, cresol purple, cresol red, cresol, cresolphthalein, cresolphthalein complexone, crystal violet, curcumin, darrow red, diaminoacridine hemisulfate, diazo red RC, dibromofluorescein, dichlorofluorescein, dichloroindophenol, dicinnamalactone, diethylaminomethyl coumarin, diethyloxacarbocyanine iodide. diethylthiatricarbocyanine iodide, dihydroxy benzenesulfonic acid, dilithium phthalocyanine, dimethyl methylene blue, dimethylglyoxime, dimethylindoaniline, dinitro diphenylamine, diphenylthiocarbazone, direct blue 71, direct green 6, direct red 23, direct red 75, direct red 81, direct violet 51, direct yellow 62, disodium phthalocyanine, disperse blue 14, disperse blue 3, disperse orange, disperse orange 11, disperse orange 25, disperse yellow 7, emodin, eosin B, eosin Y, eriochrome black T, eriochrome blue black B, erioglaucine, erythrosin B, ethyl eosin, ethyl orange, ethyl red, ethyl violet, Evans blue, fast black, fast blue B salt, fast blue BB, fast blue RR, last blue RR salt, fast corinth V salt, fast garnet GBC base, fast green FCF, fast red aluminum sall, fast red violet LB salt, fast violet B salt, fat brown RR fat green GDC salt, flavazin I, fluorescein, fluorexon, gallocyanine, guinea green B, hematoxylin, hydroxy naphthol blue, 1,4-hydroxy-naphthoquinone, indigo, indigo carmine, indoline blue, iron(II) phthalocyanine, janus green B, lacmoid, leishman stain, leuco crystal violet, leucomalachile green, leucoquinizarin, light green SF yellowish, lissamine green B, litmus, luxol last blue, malachile green base, malachite green hydrochloride, malachite green oxalate, metanill yellow, methyl eosin, methyl green, methyl orange, methyl red, methyl violet 2B. methyl violet B base, methyl yellow, methylene blue, methylene green, methylene violet 3RAX, methylesculetin, methylthymol blue, mordant blue 9, mordant brown 24, mordant brown 4, mordant orange, mordant orange 1, mordant orange 6, mordant red 19, mordant yellow 10, morin hydrate, murexide, naphthochrome green, naphthol AS, naphthol blue black, naphthol green B, naphthol yellow, naphtholbenzein, naphtholbenzene, naphtholphthalein, neutral red, new coccine, new fuchsin, new methylene blue N, nigrosin. Nile blue A, Nile blue chloride, nitrazine yellow. nitro red. nitro-phenanthroline, nitrophenol-2, nitrophenol-3, nitrophenol-4, nitrophenylazo-resorcinol, nuclear fast red. oil blue N, oil red EGN. oil red O. orange G, orange II, palatine chrome black 6BN. palaline fast yellow BLN. pararosaniline acetate, pararosaniline base, pararosaniline chloride. patent blue VF, pentamethoxytriphenylmethanol, phenanthroline, phenazine. phenol red, phenolphthalein, phenolphthalein diphosphate, phenothiazine, phenylazoandine, phenylazodiphenylamine, phenylazoformic acid. phenylazophenol phloxine B, phthalocynine, pinacyanol chloride. plasmocorinth, ponceau S, primuline, procion red MX-5B. procion yellow H-E3G. pnissian blue. purpurin, pyridlazo naphthol, pyridylazoresorcinol sodium salt, pyrocatechot violet, pyrogallol red, pyronin B. quinaldine red. quinizarin. quinoline yellow. reactive black 5. reactive blue 15, reactive blue 2, reactive blue 4, reactive orange 16, resazurin, resorcin crystal violet, rhodamine B, rhodamine B base, rhodamine GG, rhodamine S, rhodanine. rosalic acid, rose bengal, rose bengal iactone, safragine O, solvent blue 35, solvent blue 59, solvent green 3, styryl 7, sudan black B, sudan orange G, sudan red 7B, sulfobromophthalein sodium salt, sulforhodamine B. tartrazine, tetrabromophenol blue, tetrabromo phenolphthalein, tetrabromo phenolphthalein, tetraiodo phenolphthalein, tetraphenylbutadiene, tetrazolium violet, thiazol yellow G, thioflavin S, thioflavin T, thionin, thymol blue. thymolphthalein, thymolphthalein monophosphate, thymolphthalein monophosphate. toluidine blue O, triphenylmethyl bromide, tropaelin O, trypan blue, turmeric. vanillin azine. variamine blue RT salt. variamine blue RT salt, victoria blue B. victoria blue B. victoria pure blue BO, wright stain, xilidine ponceau 2R.. xylenol blue, and xylenol orange.

Some of these dyes are fluorescence dyes and there was a change in fluorescence. The reduced and oxidized forms of the above dyes can also be used.

**Indicators:** Any material, which undergoes a color change with the reaction product of ethylene oxide and ETO activator. can be used an ETO indicator. Most preferred classes of ETO indicators are dyes. pigments and their precursors. The use of a pH sensitive dye will depend upon the pH of the medium. Some dyes do not change color in ECO01270 while they do change color in polyvinylalcohol under the identical conditions. The pH of the dry coating of EC001270 is about 5 while that of polyvinylalcohol is about 7.

**Activators:** Any compound of the groups consisting of organic and inorganic salts, metal chelates and metal complexes, having a monovalent cation, reactable with ethylene oxide, to cause a rise in pH said rise in pH causing said indicator to undergo said color change, can be used as ETO activator.

Alkali metal salts, such as sodium thiocyanate and quarternary ammonium salts, such as TEAB were eltective activator. ETO activators, such as NaSCN, tetrabutylphosphonium bromide, tetrabutylphosphonium bromide and TEAB, are also referred to as activators herein. Production of indicator reactive species, such as a base, can introduce a color change in a pH-sensitive dye. A variety of classes of organic and inorganic compounds were explored as activators for monitoring ETO. They include organic inorganic salts, such as bisulfites, bisulfates, carbonates, carbamates, carboxylates, cyanates, halides, nitrites, nitrates, phenolates, phosphates, sulfates, sulfides, sulfites, and thiocyanates Organic and inorganic salls, especially halides and thicyanates of (1) alkali metals and (2) organic ammonium, sulfonium and phosphonium were more effective activators. Salts of acids with amines, such as tetramethylhexanediamine hydrobromide and tetramethylhexanediamine acetate were also effective ETO activators. Metal chelates and complexes, such as sodium acetylacetonates were also effective ETO activators.

The specific examples of compounds explored as ETO activators with some selected dyes (e.g., bromophenol blue and bromocresol purple) include aluminum acetylacetonate, aluminum ammonium sulfate, aluminum chloride, aluminum sulfate, ammonium acetate, ammonium bisulfite, ammonium bromide, ammonium carbamate, ammonium nitrate, ammonium sulfamate, ammonium sulfite, ammonium thiocyanate, ammonium thiosulfate, calcium ferrocyanide, copper thiocyanate, iron acetylacetonate, iron complexes such as potassium ferrocyanide, iron sulfate, sodium acetylacetonate, sodium bisulfite, sodium cyante, sodium diethyldithio carbamate, sodium diethyldithiocarbamate, sodium dithionite, sodium hydrosulfide. sodium nitrite, sodium persulfate, sodium sulfite, sodium sulfite, sodium sulfite, sodium sulfite, sodium thiocyanate, sodium thiocyanate, sodium thiosulfate, sulfosalicyclic acid, and tetrabutylphosphonium bromide. The activators also included a variety of primary, secondary and tertiary, aliphatic and aromatic amines neutralized with organic and inorganic acids.

**Effect of halides:** Many organic and inorganic halides are highly sensitive indicator activators. These halides include, acetyl choline chloride, ammonium bromide, choline chloride, choline iodide, dodecyltrimethylammonium bromide, glycidil trimethyl ammonium chloride, potassium bromide, potassium iodide, sodium iodide, tetrabutyl ammonium iodide, tetraethyl ammonium bromide, tetrahexyl ammonium bromide, tetramethyl ammonium chloride and tetrabutyl phosphonium bromide. Generally iodide were more bromides and bromides were more effective than chlorides.

Acid base salts, eg., those produced by neutralizing, primary, secondary and tertiary amines (e.g., hexyl amine, diethanolamine, tetramethythexane diamine) with acids such as acetic acid, hydrochloric acid, hydrobromic acid and hydroiodic acid are also effective activators.

The time required for the color change can be controlled by using a proper mixture of the activators. In order to minimize effect of ethylene oxide lower concentration of halides is preferred for certain dyes.

Activators could be polymeric, such as polyacrylic acid co-polyallylammonium chloride and polybrene.

The time required for the color change can be varied by selecting a proper mixture of halides, thiocyanates, acetates, citrates etc.

**Effect of concentration of activator:** With certain dyes as low as 0.5% of TEAB and sodium iodide was effective in introducing a noticeable color change with ETO. The rate of the color change increases with increasing the concentration of the activator. One can use 0.5 to 50 w/w% concentration of an activator. Preferred concentration range is 2-20 w/w% of the total solid.

**Polymers used as binders:** A matrix or a medium in which the activators, indicators and additives can be dissolved or dispersed are referred herein as an ETO binder. ETO binders are also referred herein to as binders, polymers or polymeric binders. A wide variety of polymeric materials can be used as binders for the indicator as long as the activators and indicators can be dissolved or dispersed in them. Both aqueous and non-aqueous binders can be used. Though one can use water-soluble, water-dispersible and water-insoluble polymers as binders for the indicator, it is preferred to use water-soluble and water-dispersible polymers as binders. The binders can be formulated in form ink formulations, such as flexo and gravure inks. Other inks such as those for letter press, offset and screen printing can also be used. Selection of the polymer depends upon the printing/coating equipment to be used.

Usually acrylic polymers, emulsion of acrylic polymers, occasionally natural polymers, such as starches, lignins, and lignin derivatives are used as binders for inks. Resins are water soluble or emulsifiable through neutralization with basic compounds, such as ammonia and amines, Inks contain a variety of additives to eliminate foaming, dispersion of pigments, rheological modifiers, and slip agents.

Polymeric binders for inks include homopolymers, copolymers and block-copolymers including those of ethylene acrylic acid, ethylene methacrylic acid, ethylene n-butyl acrylate, and ethylene methyl acrylate. Binders for inks could also be a mixture of homo and copolymers, e.g., those of methylmethacrylate, acrylic acid, styrene, methyl acrylate, other esters and crosslinking agents, such as polyvinylazaridine. Also included are polymers of acrylates, acrylic acid, acrylamide, vinyl acetate, vinyl alcohol, vinyl chloride, polyurethanes, cellulose nitrate, carboxymethyl cellulose or a mixture thereof. Desirably, the polymer is an acrylate polymer, cellulose nitrate or carboxymethylcellulose

Commercial sources for suitable polymers for ink fonnulations include Air products (Allentown, PA), Rohm and Haas (Philadelphia, PA), S.C. Johnsons and Sons (Racine, WI), Witco (Houston, PA) and ESI (Valley Stream, NY). Though a large number of polymers are suitable an ink extender. EC001270 made by Environmental Inks and Coating Co., Lithicam, MD which is composed about 40% styrene-acrylic polymers, a few percent ammonium hydroxide, additives, such as wax and alcohol and the balance water, has been found very suitable.

The nature of the binder, e.g., pH and permeability to the gases, plays an important role. Color change of bromophenol blue and TEAB system with ETO is slower in EC001270 (an acrylate) than that in polyurethane (Witcobond 213, Witco Corporation, Houston, TX) and polyvinylalcohol. Permeability of the reactive gases can also be controlled by addition of a crosslinking agent and other additives, such as plasticizers which change the permeability of ETO.

Though aqueous ink or coating formulations are preferred, one can use solvent based coating formulations polymers used in such formulations are cellulose nitrate, carboxymethyl cellulose. polyolefins, polyvinylchloride, polyurethane, polysilicones and polyepoxy.

**Binder prepared by UV curing:** As an alternative to the aforesaid binders, one can use ink and coating formulation curable with UV light. UV curable ink and coating. formulations include UV polymerizable/curable compounds such as epoxy-acrylate, polyester acrylates, and resins; typically the acrylates of diphenylol propane di-glycidyl ethers, as their principal component. In order lower viscosity and to provide a bridge between large polymer molecules, acrylic monomers are used, typically the acrylate esters of polyfunctional alcohols or glycols. The use of monomers as crosslinking agents is vital to the rapid formation of cured films, and has a major influence on the properties of both the ink or coating, and the cured product. Printing inks with epoxy-acrylate resins as their main component are usually fast curing. In order to prepare the device, one can dissolve or disperse, the indicator, activator, and additives in the UV curable extender followed by coating on substrate and curing with UV light.

**UV absorber:** If an indicator formulation is sensitive to ultraviolet light, UV absorbing materials can be added to minimize the effect. A large number of UV absorbers are available commercial, e.g., those used in sun-tan lotions, e.g., Tinuvins of Ciba-Geigy Corporation. UV absorbers include compounds such as maleic acid, sodium salicylate, benzophenone, or benzophenone tetracarboxylate and a large number of polyaromatic compounds. These UV absorbers cann't be used where the polymer for binding is prepared by UV curing.

**Effect of topcoat:** The sterilization of an article will also depend on diffusion of ETO through the binder. Hence, the time required for the color change of the device can be increased by applying a barrier coat on the device. A barrier coat can preferably be a polymeric material. The preferred barrier coat is a lacquer or an ink without pigment. The barrier coat can be a polymer listed herein. The general classes of polymers suitable for a barrier coat include resins, such as epoxy, phenol-formaldehyde, amino-formaldehyde, polyamides, vinyls, acrylics, polyurethanes, polyesters, water-soluble resins, alkyds, elastomers, and rosins. Preferred material for topcoat is a halopolymer through which ETO can diffuse slowly.

**Two-layer device:** The device can have more than one indicator layers each containing indicator, activator and binders. In order to get more than one color change at least the indicator should be different in different indicator layers and should undergo different color changes.

Both layers do not have to undergo color changes with ETO. Even if one layer undergoes a color change, a color change can be noticed, especially if the top layer becomes colorless.

**Mixtures:** Desired colors and color changes can be obtained by mixing proper dyes in appropriate amounts Similarly, the time required the color change can be varied by using a proper mixture of the activators and additives in appropriate amounts. The desired colors and the time required for the color changes can be obtained by selecting a proper mixture of compatible binders, additives and activators.

**Substrate:** Though the device could be a self-supporting polymer film containing the activator and indicator, it is desirable to prepare the device on substrate. The device can also be made by coating the indicating formulation on a substrate. The substrate could be any solid surface; for example, that made from paper, plastic, ceramic and metal. The substrate could be a container, e.g., bag or pouch, for items to be sterilized. The sterilization indicator can also be prepared in form of stickers,

Although any solid substrate having a smooth surface can be used, a preferred substrate is a flexible and transparent plastic film, and natural (cellulose) and synthetic (e.g., spun bonded polyolefins. e.g., Tyvek^{R}) papers. Plastic films, such as polyethylene, polypropylene, polyvinylchloride, polymethylmethacrylate, polyurethanes, nylons, polyesters, polycarbonates, polyvinylacetale, cellophane and esters of cellulose can be used as the transparent substrate. Metal foils, such as aluminum can be used. The most preferred substrates are the 5 - 300 microns thick films of polyethylene terephthalate, cellulose paper and Tyvek^{R}.

**pH ranges:** The pH of the system depends upon the indicator, activator, binder and additive. If the binder is acidic, e.g., polyacrylic acid or an acid as an additive, e.g., citric acid, the pH of the system will be low, e.g., pH of 2 to 3. However, on the other hand, if the basic system is used, e.g., polyethyleneimine as a binder or a base such as sodium hydroxide or an amine such as diethanolamine is added as an additive, the pH of system could be high, e.g., pH of 10 to 12. The indicator, e.g., a pH sensitive dye, to be selected would depend upon on the pH of the coating. Production of a base upon exposure to ETO would shift the pH of the system towards the basic side. The indicator should under go at least one color change above the pH of the binder system containing the indicator, activator and additive. The pH range of the system could be 3 to 11. The preferred pH range of the system is 5-9. The most preferred pH range of the system is 6-8. The change in pH of the system upon exposure to ETO could be as much as 5, e.g., from 4-9. The preferred change in pH is about 2, e.g., form 6 to 8 or 5 to 7.

**Temperature:** The indicator can undergo color change from a very low temperature (e.g., -30°C) to a very high temperature of a hundred degrees centigrade. The preferred temperature is below about 70°C, preferred temperature is 20-60°C.

**Time:** The time required for the color change can be varied by varying one or more of parameters such as thickness of the binder and the indicator layer, concentration of the activator, concentration of the indicator, concentration of other additives, nature of the binder, nature of the barrier, thickness of the barrier, nature of the activator, nature of the indicator, nature of the additives and concentration of ETO. For example, the time required for the color change can be increased by (1) decreasing the concentration of activator (2) selecting a slow activator, (3) additives which neutralize the reaction products, such as an acid or phenols and (4) selecting an indicator having higher pH range for the color change. Similarly, the time required for the color change can be decreased by (1) increasing the concentration of activator (2) selecting a faster activator, (3) additives, such as an amine, and (4) selecting an indicator having lower pH range for the color change.

For a given sterilization cycle, the time required for the color change can be varied by varying one or more of the following parameters (w/w = weight to weight percent):

### 1. Thickness of the polymer indicator layer.

The thickness of the indicator and barrier layers may vary from a micron to a few hundred microns. The preferred thickness is 1-50 microns and the most preferred range is 2-20 microns.

### 2. Concentration of the activator.

The concentration of activator may vary from 0.1 to 50 w/w%. The preferred concentration is 1 to 20 w/w% and the most preferred concentration is 2-10 w/w/%.

### 3. Concentration of the indicator.

The concentration of the indicator may vary from 0.1 to 20w/w%. The preferred concentration is 1 to 10w/w% and the most preferred concentration is 2-5 w/w%.

### 4. Concentration of other additives.

The concentration of additives may vary from 0.1 to 20w/w%. The preferred concentration is 0.5 to 10w/w% and the most preferred concentration is 1-5w/w/%.

### 5. Nature of the polymer.

### 6. Nature of the barrier.

This is the same group as the polymer, but may be different from it

### 7. Thickness of the barrier.

The barrier may be between 2 and 200 microns thick, preferrably 2-20 microns.

### 8. Nature of the activator.

### 9. Nature of the indicator.

### 10. Nature of the additives.

### 11. Concentration of ETO:

The time required for the color change will depend upon concentration of ETO gas. ETC gas can be diluted with gases such as air, nitrogen, argon, carbon dioxide, tluorocarbons and water vapor (humidity). The time required for the color change will be shorter with higher concentration of ETO and vice versa. Higher humidity will decrease the time required for the color change.

The preferred time range for sterilization is from 10 minutes to 10 hours, The most preferred time is about 30 minutes to five hours.

**Monitoring ETO:** In order to protect workers who use ETO while on the job, the Occupational Safety and Health Administration (OSHA) has established a limit of 1 ppm in workplace air for an 8-hour workday and a limit of 5 ppm for a 15-minute period. A filter paper coated with a mixture of bromothymol blue and potassium iodide changes from faint yellow to green-blue within minutes at about 100 parts per million.

The device can be used for monitoring low concentration of ETO. Though we demonstrated the concept with ETO, the device can also be used with other epoxides.

**Advantages:** The device offers some of the major advantages.
* . The formulations are inexpensive.
* . The ingredients are considered nontoxic.
* . It is easy to make the ink formulations, just by mixing proper ingredients in an ink extender.
* . The device is selective to ETO. It is unaffected by steam, heat and radiation.
* . It is unaffected by sealing hot bar.
* . It has required pot life.
* . There is no bleeding/diffusion of dyes.
* . The ingredients (indicators/dyes and activators/additives) are water soluble. No grinding of ingredients required.
* Ink is printable with gravure and flexo presses on polyester, paper and Tyvek®.
* The print rolls are easy to clean.
* The time required for the color change can be varied by simple means.
* It provides desired color changes (from a starting light color, such as white/colorless, yellow, orange, pink, or red to a final dark color, such as blue, green, black, purple or violet).
* It provides an intermediate color for monitoring a partial cycle

### EXAMPLES

### Example 1. General procedure for preparation of the sample devices.

In a 10ml test tube were added about 3 ml of EC001270 (an acrylate ink extender supplied by Environmental Inks and Coating, Co, Lithicum, MD), about 0.5 ml of an activator solution (e.g., 50w/w% TEAB in water) and about 0.5 ml of an indicator solution (e.g., 4 w/w% solutions of a dye, e.g., bromocresol purple in ethanol). The contents were mixed and coated with either #5 or #10 wire wound rod on a 100 micron polyester film and paper. The coatings were dried in an oven at about 50°C for about a few minutes.

### Example 2. Exposure to ETO.

Samples of example 1 were placed in cell (e.g., 30 x 30 x 1 cm³) and flushed with 100% ETO gas for a few minutes. The color changes of the samples were noted.

Some selected samples were also exposed to 10% ETO (90% a chlorofluoro gas) at 140°F, 100% humidity.

### Example 3: Effect of activator on dyes.

Using the general procedure described in example 1, coatings were prepared from EC001270 as a binder, TEAB as an activator, and most of the dyes listed in Table 1 as indicators. The coatings were exposed to ETO for a few hours. Some representative color changes are listed Table 2.

**Table 2.**

| Representative color changes of some dyes with EC001270 and TEAB | | |
|---|---|---|
| Dye | Original color | Color after ETO exposure |
| Victoria Blue B | Blue | Brown |
| Leuco Crystal Violet | Blue | Colorless |
| Basic blue 66 | Blue | Red |
| Naphtholbenzene alpha | Brown | Green |
| Naphtholphthalein-alpha | Colorless | Green |
| Lissamine Green B | Gray | Clear |
| Brilliant Green | Green | Colorless |
| Guinea Green B | Green | Colorless |
| Chromoxane Cyanine R | Lavender | Yellow |
| Quinizarin | Peach | Lavender |
| Rosalic Acid | Peach | Pink |
| Alizarin Red | Peach | Purple |
| Acid Fuschin | Pink | Colorless |
| Acid Red 1 | Pink | Red |
| Methyl Red | Pink. | Yellow |
| Palatine chrome black 6BN | Purple | Blue |
| Bromocresol Purple | Yellow | Blue |
| Bromophenol Blue | Yellow | Blue |
| Bromothymol Blue | Yellow | Blue |
| Thiazol Yellow G | Yellow | Brown |
| Cresol Red | Yellow | Purple |
| Alizarine Yellow R | Yellow | Red |
| Phehol Red | Yellow | Red |

The color change usually occurs selectively with ETO. There is little effect of steam and radiation.

Similar color changes were obtained with many other activators, such as sodium thiocyanate, and tetrabulylphosphonium bromide, and binders e.g., polyurethane (Witcobond 213, Wilco Corporation, Houston, TX) and cellulose nitrate.

### Example 4. Effect of other halides.

Using the procedure described in example 1, coatings were prepared with some organic and inorganic halides as activators and bromophenol blue and bromocresol purple as indicators. The coatings were exposed to ETO. All coatings changed from yellow to blue. Iodide and bromides were more effective than other halides in introducing the color change.

The halide tried included, acetyl choline chloride, ammonium bromide, barium bromide, calcium bromide, choline chloride, choline iodide, dimethyldioctadecylammonium bromide, diphenyliodinium bromide, dodecyltrimethylammonium bromide, glycidil trimethyl ammonium chloride, iron (III) bromide, phenacylpyridinium bromide, potassium bromide, potassium bromide, potassium iodide, sodium iodide, tetrabutyl ammonium iodide, tetrabutyl phosphonium bromide, tetrabutylammonium bromide, tetraethyl ammonium bromide, tetrahexyl ammonium bromide, tetramethyl ammonium chloride, tetramethylhexaenediamine hydrobromide, tetramethylhexanediamine hydroiodide, and zinc bromide.

### Example 5. Device without binder.

Fifty (w/w) percent solutions of tetrabutylphosphonium bromide (PB), sodium thiocyanate (NaSCN) and tetraethylammonium bromide (TEAB) were prepared in water. Pieces of filter paper were dipped in the solutions. Drops of a universal pH indicator (Fisher Scientific) were placed on the coated paper filter papers. The filter papers were dried in an oven al 50°C for 1 hour. The filter papers were then exposed to ETO gas. The color change occurred within seconds when exposed to ETO. The color change was fastest with sodium thiocyanate and slowest with tetraethyl ammonium bromide. The color changes are reported below:

**Table 3.**

| The color changes of a universal pH indicator with different activators. | | | |
|---|---|---|---|
| Color | PB | NaSCN | TEAB |
| Original | Red | Red | Yellow |
| Exposed | Green | Violet | Green |

### Example 6. Detection of residual absorbed ETO.

A piece of 100-micron polyester film having a coating of EC001270 was exposed to ETO for ten minutes. The coated film was removed and hang In air for ten minutes. A coating of EC001270, bromophenol blue and TEAS on polyester was placed on ETO exposed film. The yellow coating turned green within 5 minutes.

### Example 7. Mixture of two indicators.

Using the procedure described in example 1. a coating was prepared using ethyl red, bromophenol blue and their 1:1 mixture as indicators and TEAB as an activator. The ethyl red coating changed from red-to-yellow, that of bromophenol blue changed from yellow-to-green-to-blue and that of their mixture changed from orange -> yellow -> yellow green -> green.

### Example 8.

Using the procedure described in example 1, a coating was prepared using bromophenol blue as an indicator and 1:1 mixture of TEAB and NaSCN as activators. The coaling was yellow. The coating changed from yellow-to-green-to-blue upon exposure to ETO.

### Example 9. Pilot coating.

In 13kg EC001270 extender the following were added while stirring: 200g of 28% ammonium hydroxide, 3kg of water. 2kg of tetraethylammonium bromide, 2kg of sodium thiocyanate. 100g of bromocresol purple, and 135g of bromothymol blue. The formulation was coated on paper at Rexam Medical Packaging. Mundelein IL.

The samples were exposed to 100% ETO. The indicator changed from yellow-to-green-to-blue. The indicator has very little effect of (1) dry heat at 60°C five days, (2) 100% relative humidity at 60°C for five days, (3) normal steam sterilization treatment (121°C. 30 minutes). The final blue color was essentailly unaffected after 5 days at 50°C at 100%RH.

Clearly it should now be quite evident to those skilled in the art, that while this invention has been shown and described in detail in the context of a preferred embodiment, and with various modifications thereto, a wide variety of other modifications can be made without departing from scope of the inventive teachings.

## Claims

1. A process using a device for monitoring sterilization of materials, said device comprising
at least one layer of polymer, having incorporated therein
a) an indicator capable of undergoing at least one color change when subjected to a rise in pH,
b) an activator for said indicator, said activator being a compound of the group consisting of organic and inorganic salts, metal chelates and metal complexes, having a monovalent cation, readable with ethylene oxide, to cause a rise in pH said rise in pH causing said indicator to undergo said color change, comprising the steps of
c) affixing the device to said materials or containers containing same
d) carrying out the process of sterilization including the step of introducing ethylene oxide and
e) observing the presence of a color change of said device.

2. A process of using a device for monitoring ethylene oxide said device comprising
at least one layer of polymer, having incorporated therein
a) an indicator capable of undergoing at least one color change when subjected to a rise in pH,
b) an activator for said indicator, said activator being a compound of the group consisting of organic and inorganic salts, metal chelates and metal complexes, having a monovalent cation, reactable with ethylene oxide, to cause a rise in pH said rise in pH causing said indicator to undergo said color change, comprising the steps of
c) exposing the device to ethylene oxide
d) observing the presence of color change in the device.

3. The process of claim 1 or 2 wherein the cation is selected from the group consisting of lithium, sodium, potassium, cesium, quartenary nitrogen, quarternary phosphorous and organic quarternary sulfur.

4. The process of any of claims 1 to 3 wherein the anion of the salt is selected from the group consisting of bisulfite, bisulfate, carbonate, carbamate, carboxylate, cyanate, halide, nitrite, nitrate, phenolate, phosphate, sulfate, sulfide, sulfite and thiocyanate.

5. A method for monitoring sterilization with ethylene oxide comprising:
exposing a monitor composition to ethylene oxide comprising at least one layer of polymer, having incorporated therein
a) an indicator capable of undergoing at least one color change when subjected to a rise in pH
b) an activator for said indicator, said activator being a compound of the group consisting of organic and inorganic salts, metal chelates and metal complexes, having a monovalent cation, reactable with ethylene oxide, to cause a rise in pH said rise in pH causing said indicator to undergo said color change when exposed to ethylene oxide and noting the change in color of said indicator

6. The method of claim 5 wherein the cation is selected from the group consisting of lithium, sodium, potassium, cesium, quarternary nitrogen and quarternary phosphorus.

7. The method of claim 6 wherein the anion is selected from the group consisting of bisulfite bisulfate, carbonate, carbamate, carboxylate, cyanate, halide, nitrite, nitrate, phenolate, phosphate, sulfate, sulfide, sulfite and thiocyanate.

## Patentansprüche

1. Ein Verfahren unter Verwendung einer Vorrichtung zum Überwachen der Sterilisierung von Materialien, wobei die Vorichtung umfaßt:
wenigstens eine Schicht eines Polymers, die beinhaltet:
a) einen Indikator, der in der Lage ist, wenigstens eine Farbveränderung aufzuzeigen, wenn er einem pH-Wert-Anstieg ausgesetzt ist,
b) ein Aktivator für diesen Indikator, wobei der Aktivator eine Verbindung ist, ausgewählt aus der Gruppe bestehend aus organischen und anorganischen Salzen, Metallchelaten und Metallkomplexen, mit einem monovalenten Kation, die mit Ethylenoxid reagiert, um einen pH-Anstieg zu verursachen, wobei dieser pH-Anstieg verursacht, daß der Indikator die Farbveränderung vornimmt, umfassend die Schritte
c) Anbringen der Vorrichtung auf diesen Materialien oder Behältern, die diese enthalten,
d) Ausführen des Verfahrens der Sterilisierung, einschließlich des Schrittes des Einführens von Ethylenoxid und
e) Beobachten der Gegenwart einer Farbveränderung der Vorrichtung.

2. Ein Verfahren zur Verwendung einer Vorrichtung zum Überwachen von Ethylenoxid, wobei die Vorrichtung umfaßt:
wenigstens eine Polymerschicht, die beinhaltet
a) einen Indikator, der in der Lage ist, wenigstens eine Farbveränderung vorzunehmen, wenn er einem pH-Anstieg ausgesetzt wird,
b) einen Aktivator für diesen Indikator, wobei der Aktivator eine Verbindung ausgewählt aus der Gruppe bestehend aus organischen und anorganischen Salzen, Metallchelaten und Metallkomplexen mit einem monovalenten Kation ist, die mit Ethylenoxid reagiert, um einen pH-Anstieg zu verursachen, wobei der pH-Anstieg verursacht, daß der Indikator die Farbveränderung vornimmt, umfassend die Schritte:
c) Aussetzen der Vorrichtung gegenüber Ethylenoxid
d) Beobachten der Gegenwart von Farbveränderungen in der Vorrichtung.

3. Das Verfahren nach Anspruch 1 oder 2, wobei das Kation ausgewählt ist aus der Gruppe bestehend aus Lithium, Natrium, Kalium, Cäsium, quartärem Stickstoff, quartärem Phosphor und organischem quartären Schwefel.

4. Das Verfahren nach einem der Ansprüche 1 bis 3, wobei das Anion des Salzes ausgewählt ist aus der Gruppe bestehend aus Bisulfit, Bisulfat, Carbonat, Carbamat, Carboxylat, Cyanat, Halogenid, Nitrid, Nitrat, Phenolat, Phosphat; Sulfat, Sulfid, Sulfit und Thiocyanat.

5. Ein Verfahren zum Überwachen der Sterilisation mit. Ethylenoxid:
Aussetzen einer Überwachungszusammensetzung gegenüber dem Ethylenoxid, die wenigstens eine Schicht eines Polymers umfaßt, die beinhaltet:
a) einen Indikator, der in der Lage ist, wenigstens eine Farbveränderung vorzunehmen, wenn er einem pH-Anstieg ausgesetzt wird
b) einen Aktivator für diesen Indikator, wobei der Aktivator eine Verbindung aus der Gruppe bestehend aus organischen und anorganischen Salzen, Metallchelaten und Metallkomplexen mit einem monovalenten Kation ist, die mit Ethylenoxid reagiert, um einen pH-Anstieg zu verursachen, wobei der pH-Anstieg verursacht, daß der Indikator die Farbveränderung vornimmt, wenn er gegenüber Ethylenoxid ausgesetzt ist, und
Beobachten der Farbveränderung des Indikators.

6. Das Verfahren nach Anspruch 5, wobei das Kation ausgewählt ist aus der Gruppe bestehend aus Lithium, Natrium, Kalium, Cäsium, quartärem Stickstoff und quartärem Phosphor.

7. Das Verfahren nach Anspruch 6, wobei das Anion ausgwählt ist aus der Gruppe bestehend aus Bisulfit, Bisulfat, Carbonat, Carbamat, Carboxylat, Cyanat, Halogenid, Nitrid, Nitrat, Phenolat, Phosphat, Sulfat, Sulfid, Sulfit und Thiocyanat.

## Revendications

1. Procédé utilisant un dispositif pour le monitorage de la stérilisation de matériaux, ledit dispositif comprenant
au moins une couche de polymère, dans laquelle sont incorporés
a) un indicateur capable de subir au moins un changement de couleur lorsqu'il est soumis à une augmentation de pH,
b) un activateur pour ledit indicateur, ledit activateur étant un composé du groupe constitué de sels organiques et inorganiques, de chélates métalliques et de complexes métalliques, ayant un cation monovalent, pouvant réagir avec de l'oxyde d'éthylène pour provoquer une augmentation de pH, ladite augmentation de pH entraînant ledit changement de couleur dudit indicateur, comprenant les étapes
c) de fixation du dispositif auxdits matériaux ou aux récipients les contenant
d) de mise en oeuvre du procédé de stérilisation comprenant l'étape d'introduction de l'oxyde d'éthylène et
e) d'observation de la présence d'un changement de couleur dudit dispositif.

2. Procédé d'utilisation d'un dispositif pour le monitorage de l'oxyde d'éthylène, ledit dispositif comprenant
au moins une couche de polymère, dans laquelle sont incorporés
a) un indicateur capable de subir au moins un changement de couleur lorsque soumis à une augmentation de pH,
b) un activateur pour ledit indicateur, ledit activateur étant un composé du groupe constitué de sels organiques et inorganiques, de chélates métalliques et de complexes métalliques, ayant un cation monovalent, pouvant réagir avec de l'oxyde d'éthylène pour provoquer une augmentation de pH, ladite augmentation de pH entraînant ledit changement de couleur dudit indicateur comprenant les étapes
c) d'exposition du dispositif à de l'oxyde d'éthylène
d) d'observation de la présence d'un changement de couleur dans le dispositif

3. Procédé selon la revendication 1 ou 2, dans lequel le cation est choisi dans le groupe constitué de lithium, sodium, potassium, césium, azote quaternaire, de phosphore quaternaire et de soufre quaternaire organique.

4. Procédé selon l'une quelconque des revendications 1 à 3, dans lequel l'anion du sel est choisi dans le groupe constitué de bisulfite, bisulfate, carbonate, carbamate, carboxylate, cyanate, halogénure, nitrite, nitrate, phénolate, phosphate, sulfate, sulfide, sulfite et de thiocyanate.

5. Méthode de monitorage de la stérilisation avec de l'oxyde d'éthylène comprenant :
l'exposition à de l'oxyde d'éthylène d'une composition de monitorage comprenant au moins une couche de polymère, dans laquelle sont incorporés
a) un indicateur capable de subir au moins un changement de couleur lorsque soumis à une augmentation de pH,
b) un activateur pour ledit indicateur, ledit activateur étant un composé du groupe constitué de sels organiques et inorganiques, de chélates métalliques et de complexes métalliques, ayant un cation monovalent, pouvant réagir
avec de l'oxyde d'éthylène pour provoquer une augmentation de pH, ladite augmentation de pH entraînant ledit changement de couleur
lorsqu' exposé à de l'oxyde d'éthylène et
l'observation du changement de couleur dudit indicateur

6. Méthode selon la revendication 5 dans laquelle le cation est choisi dans le groupe constitué de lithium, sodium, potassium, césium, azote quaternaire et phosphore quaternaire.

7. Méthode selon la revendication 6 dans laquelle l'anion est choisi dans le groupe constitué de bisulfite, bisulfate, carbonate, carbamate, carboxylate, cyanate, halogénure, nitrite, nitrate, phénolate, phosphate, sulfate, sulfide, sulfite et thiocyanate.
